# EUROPÄISCHE PATENTSCHRIFT

(11) **EP 2 727 979 B1**
(45) Veröffentlichungstag und Bekanntmachung des Hinweises auf die Patenterteilung: **07.01.2015**
(21) Anmeldenummer: 12191124.2
(22) Anmeldetag: 02.11.2012
(51) Int. Cl.: B01D 53/22, C07C 1/04, C01B 3/50, C10G 2/00

(54) **Fischer-Tropsch-Verfahren zur Herstellung von Kohlenwasserstoffen aus Biogas**
Fischer-Tropsch method for producing hydrocarbons from biogas
Procédé Fischer-Tropsch destiné à fabriquer des hydrocarbures à partir de biogaz

(43) Veröffentlichungstag der Anmeldung: 07.05.2014
(73) Patentinhaber: Helmholtz-Zentrum Geesthacht Zentrum für Material- und Küstenforschung GmbH, 21502 Geesthacht (DE)
(72) Erfinder: Brinkmann, Torsten, 21502 Geesthacht (DE)
(74) Vertreter: UEXKÜLL & STOLBERG

(56) Entgegenhaltungen:
- WO-A1-2007/077138
- WO-A1-2011/141635
- CN-A- 101 434 507
- US-A1- 2011 203 455
- US-B1- 6 495 610

## Beschreibung

Die vorliegende Erfindung betrifft ein Verfahren zur Herstellung von Kohlenwasserstoffen mit einem Fischer-Tropsch-Verfahren.

### Stand der Technik

Die Vergasung von Biomasse für die Erzeugung eines gasförmigen Einsatzstroms für nachfolgende chemische Synthesen gewinnt immer mehr an Bedeutung. Eine dieser Synthesen ist die Fischer-Tropsch Synthese. Mit der Fischer-Tropsch Synthese werden aus einem Wasserstoff und Kohlenmonoxid enthaltenden Einsatzgas (Synthesegas) höhere Kohlenwasserstoffe wie Ethylen, Propylen, flüssige Kraftstoffe oder Wachse erzeugt.

Die erzielbare Ausbeute an höheren Kohlenwasserstoffen wie C₅₋₂₀-Kohlenwasserstoffen und leichteren Kohlenwasserstoffen wie C₂₋₄-Kohlenwasserstoffen als Einsatzstoffe für die chemische Industrie wird durch die erforderliche Ausschleusung von CO₂ zur Erhöhung des H₂/CO-Verhältnisses im Synthesegas, den Energiebedarf und die erreichten Umsätze und Selektivitäten bei den einzelnen Verfahrensstufen wesentlich bestimmt. Diese Trennaufgaben sind in der Regel energetisch und apparativ aufwändig und wirken sich negativ auf die technische Umsetzung von Biomassenvergasung im größeren Stil aus.

Neben Wasserstoff und Kohlenmonoxid enthält ein durch Biomassenvergasung hergestelltes Synthesegas mindestens noch Methan, Kohlendioxid und Wasser. Die Einstellung des Verhältnisses der Einsatzströme an Kohlenmonoxid und Wasserstoff erfolgt gemäß dem Stand der Technik über Methanreformierung, Wassergas-Shiftreaktionen und Kohlendioxidabtrennung. Ferner ist eine Wasserabtrennung notwendig (M.J.A. Tijmensen et al., Biomass and Bioenergy 23 (2002) 129-152). Die Reaktionsverfahren sind apparativ aufwändig. Selbiges gilt für Verfahren zur Gastrocknung und Kohlendioxidabtrennung. Diese werden durch physikalische oder chemische Absorptionsverfahren realisiert. Nachteilig bei diesen Verfahren ist ferner die Verwendung eines Trennhilfsstoffes, welcher durch einen zusätzlichen Prozessschritt energieaufwändig aufbereitet werden muss.

Nicht abreagierter Wasserstoff wird vorteilhaft in den Reaktor zurückgeführt. Hierzu kann der Wasserstoff mittels Druckwechseladsorptionsverfahren abgetrennt werden. Nachteilig ist bei beiden Verfahren die Notwendigkeit, das Gas vor Eintreten in die Trenneinheit zu kühlen.

Die Auftrennung der Produkte kann z.B. durch Tieftemperaturrektifikation bei erhöhten Drücken erfolgen. Die Einstellung dieser Betriebsbedingungen ist wiederum apparativ und energetisch aufwändig, vor allem wenn sich noch leichte Gase wie Wasserstoff, Methan oder Kohlenmonoxid im aufzutrennenden Produktgas befinden.

Etwaig vorhandenes Kohlendioxid muss vor Eintritt in die Tieftemperaturrektifikation entfernt werden. Stand der Technik sind hierfür Absorptionsverfahren mit chemisch oder physikalisch wirkenden Waschmitteln.

Die CN 101 186 550 A beschreibt ein Verfahren, bei dem zwei Fischer-Tropsch Reaktoren in Reihe betrieben werden. Die Produktströme werden in Gas-Flüssig oder Gas-Flüssig-Flüssig-Separatoren getrennt. Die die leichten Gase enthaltenen Produktströme dieser Separatoren werden den Einsatzströmen der Fischer-Tropsch Reaktoren zugemischt. Es wird keine Einstellung des Synthesegasverhältnisses beschrieben.

Die CN 101 307 245 A offenbart einen Festbettreaktor für die Fischer-Tropsch Synthese. Es wird auch auf die Rezirkulierung nicht abreagierter Einsatzgase eingegangen. Das hierfür verwendete Verfahren wird nicht offenbart.

Die CN 101 434 507A offenbart eine Kaskade bestehend aus Kondensatoren und darauf folgenden Trennbehältern, in welchen die Fischer-Tropsch Produkte aufgetrennt werden. Hierzu wird das Produktgas gekühlt, sodass eine Flüssigphase entsteht welche abgetrennt wird. Das nach der Kühlung verbleibende Gas wird weitergekühlt, sodass wieder eine abzuziehende Flüssigphase entsteht. Diese Verfahrensweise wird wiederholt, bis nur noch Wasserstoff und Kohlenmonoxid im Gas enthalten sind, welches dann rezirkuliert wird. Die entstehenden Flüssigphasen werden gemischt und einer Demethanisierungskolonne zugeführt. Das Verfahren beruht auf der Ausnutzung von Dampf-Flüssigphasengleichgewichten.

Die CN 101 559 320 A offenbart ein Verfahren, bei dem die im Reaktor produzierten Gase gekühlt und einer Demethanisierungskolonne zugeführt werden. Das im Sumpf der Kolonne abgeführte Kohlenwasserstoffgemisch wird einer weiteren Aufbereitung zugeführt. Das Kopfprodukt wird einer Absorptionsstufe zugeführt, in dem die noch enthaltenen höheren Kohlenwasserstoffe mit Hilfe einer aus höheren Kohlenwasserstoffen bestehenden Absorptionsflüssigkeit separiert werden und zum Eingang der Demethanisierungskolonne zurückgeführt werden. Die oben aus der Absorptionskolonne austretenden Gase werden abgeführt. Membranverfahren finden keine Anwendung.

Die CN 101 979 468 A offenbart die Rezirkulierung des nicht abreagierten Gases einer Fischer-Tropsch-Reaktion, die Mischung mit Kohlendioxid und die anschließende katalytische Reformierung. Dieses Gas wird gemeinsam mit dem Synthesegas dem Fischer-Tropsch Reaktor zugeführt. Die Einstellung des Synthesegasverhältnisses wird durch die erwähnte Reformierung erreicht.

Die DE 10 2010 011 076 A1 offenbart ein Verfahren, bei dem eine mittelsiedende Produktfraktion aus einem Fischer-Tropsch Reaktor gewonnen werden soll. Hierzu werden in zwei hintereinander geschaltete Gas-Flüssig-Separatoren zuerst die hochsiedenden Kohlenwasserstoffe entnommen und dem Reaktor über eine beheizte Leitung wieder zugeführt. Im zweiten Separator wird die Flüssigphase als Produkt aus dem Verfahren abgeführt und in einem Flüssig-Flüssig Separator von Wasser befreit. Der gasförmige Produktstrom wird wiederum dem Reaktor zugeführt.

Die US 4,049,741 offenbart ein Verfahren, bei dem die Fischer-Tropsch Produkte durch weitere Reaktionsschritte in ihrer Oktanzahl erhöht werden. Es werden Gasströme separiert und in den Reaktionsteil zurückgeführt.

Die WO 03/072530 A1 schlägt eine verbesserte Aufbereitung und teilweise Rückführung von Fischer-Tropsch Produkten vor. Teilweise untergehen die rückgeführten Ströme eine chemische Umsetzung.

Die WO 01/42175 A1 offenbart ein Verfahren, bei dem ein Synthesegas aus Kohlenmonoxid, Wasserstoff und Kohlendioxid erzeugt und einem Fischer-Tropsch Reaktor zugeführt wird. Das Produktgas aus dem Reaktor wird in eine höhere Kohlenwasserstofffraktion zur weiteren Verarbeitung, einer wässrigen Phase und einem zum Reaktoreingang zu rezirkulierenden Abgasstrom aufgeteilt. Der Abgasstrom wird vor Zumischung zum Reaktoreinsatzstrom noch einer Dampfreformierung zugeführt. Als Methode zur Abtrennung von Wasserstoff wird die Druckwechseladsorption erwähnt. Zur Einstellung des Kohlendioxidgehalts wird die umgekehrte Wassergasshiftreaktion erwähnt.

Die WO 2004/092306 A1 offenbart ein Verfahren, bei dem ebenfalls nicht abreagierte Einsatzgase rezirkuliert werden. Die Abtrennung der Gase erfolgt durch Druckwechseladsorption.

Die Rückführung von nicht abreagierten Wasserstoff und Kohlenmonoxid mittels Druckwechseladsoption wird auch in der FR 2 807 027 und der FR 2 891 277 vorgeschlagen.

Die WO 2005/005576 A1 offenbart, das bei der Fischer-Tropsch Synthese entstehende Abgas von Kohlendioxid zu befreien und einer energetischen Nutzung zuzuführen.

Die WO 2011/141635 A1 offenbart ein Verfahren zur Herstellung einer Kohlenwasserstoffzusammensetzung, umfassend die Schritte: Bereitstellung einer Biomasse; Vergasung der Biomasse in Gegenwart von Sauerstoff; Erhöhung der Wasserstoff-zu-Kohlenmonoxid-Verhältnis des Gases; Zuführung des Gases zu einem Fischer-Tropsch-Reaktor; Umwandlung des Gases in dem Reaktor; Gewinnung der Kohlenwasserstoffzusammensetzung.

Es besteht ein Bedarf die Erzeugung von höheren Kohlenwasserstoffen mittels Fischer-Tropsch-Synthese aus Synthesegas, welches zum Beispiel aus der Biomassenvergasung oder aus Biogas gewonnen werden kann, insbesondere in energetischer und apparativer Hinsicht weiter zu verbessern.

### Zusammenfassung der Erfindung

Die Aufgabe wird durch ein Verfahren zur Herstellung von Kohlenwasserstoffen aus Biomasse gelöst bei dem ein Einsatzstrom aus der Biomassenvergasung, der Wasserstoff, Kohlenmonoxid sowie weitere Gase und/oder Dämpfe umfasst, zunächst einer ersten Membranstufe zugeführt wird, die eine wasserdampfselektive und kohlendioxidselektive Membran umfasst und durch die mindestens ein Teil des in dem Einsatzstrom vorhandenen Wasserdampfs und ein Teil des im Einsatzstrom vorhandenen Kohlendioxids vom Einsatzstrom zur Bildung eines Wasserdampf und Kohlendioxid umfassenden Permeatstroms der ersten Membranstufe und eines Wasserstoff und Kohlenmonoxid enthaltenden Retentatstroms der ersten Membranstufe getrennt wird, der Retentatstrom der ersten Membranstufe einem Fischer-Tropsch-Reaktor zugeführt wird, in dem ein Produktstrom erzeugt wird, der Wasserstoff, Wasserdampf, Kohlenwasserstoffe sowie gegebenenfalls weitere Gase und/oder Dämpfe umfasst, der Produktstrom aus dem Fischer-Tropsch-Reaktor einer zweiten Membranstufe zugeführt wird, die eine wasserstoffselektive und wasserdampfselektive Membran umfasst und durch die mindestens ein Teil des im Produktstrom des Fischer-Tropsch-Reaktors vorhandenen Wasserstoffs und Wasserdampfs zur Bildung eines Wasserstoff und Wasserdampf enthaltenden Permeatstroms der zweiten Membranstufe und eines Kohlenwasserstoffe enthaltenden Retentatsstroms der zweiten Membranstufe getrennt werden, der Permeatstrom der zweiten Membranstufe einer Kühlung und Kondensation zur Ausschleusung des im Permeatstrom der zweiten Membranstufe enthaltenen Wassers zugeführt, rekomprimiert, und mit dem Retenatstrom der ersten Stufe vermischt wird, um dem Fischer-Tropsch-Reaktor wieder zugeführt zu werden, der Retentatsstrom der zweiten Membranstufe einer ersten Separatorstufe zugeführt wird, in der der Retentatsstrom der zweiten Membranstufe mindestens in eine Gasphase und eine flüssige Fraktion sowie gegebenenfalls in weitere Fraktionen aufgetrennt wird, die Gasphase der ersten Separatorstufe einer dritten Membranstufe zugeführt wird, die eine wasserstoffselektive und wasserdampfselektive Membran umfasst und in der mindestens ein Teil des in der Gasphase der ersten Separatorstufe vorhandenen Wasserstoffs und Wasserdampfs von leichten Gasen zur Bildung eines Wasserstoff und Wasserdampf enthaltenden Permeatstroms der dritten Membranstufe und eines leichte Gase enthaltenden Retentatstroms der dritten Membranstufe getrennt werden, und der Wasserstoff und Wasserdampf enthaltende Permeatstrom der dritten Membranstufe mit dem mittels Kondensation teilweise getrockneten Permeatstrom der zweiten Membranstufe und dem Retentatstrom der ersten Membranstufe zur Rückführung in den Fischer-Tropsch-Reaktor gemischt wird.

Durch den Einsatz einer ersten Membranstufe, die eine kohlendioxid- und wasserdampfselektive Membran umfasst, kann das Einsatzgas für eine optimale Betriebsweise des Fischer-Tropsch Reaktors konditioniert werden. Störende Gase wie Wasserdampf und Kohlendioxid werden mindestens teilweise aus dem Einsatzstrom entfernt. Die Konditionierung des Einsatzgases für die Fischer-Tropsch-Reaktion erfolgt vorzugsweise bei Temperaturen von etwa 10°C bis etwa 50°C, besonders bevorzugt bei Umgebungstemperatur von etwa 20°C bis etwa 30°C.

Nach der Fischer-Tropsch-Reaktion nicht abreagierter Wasserstoff sowie Wasserdampf werden in einer zweiten Membranstufe, die eine wasserstoffselektive und wasserdampfselektive Membran umfasst, mindestens teilweise vom Produktstrom getrennt. Als Membran der zweiten Membranstufe wird vorzugsweise eine temperaturbeständige Membran verwendet, wobei die Abtrennung von Wasserstoff und Wasserdampf bevorzugt bei Temperaturen von bis zu etwa 250°C, weiter bevorzugt bei Temperaturen von etwa 75°C bis etwa 250°C, besonders bevorzugt bei Temperaturen von etwa 100°C bis etwa 200°C erfolgt.

Eine dritte Membranstufe, die eine wasserstoffselektive und wasserdampfselektive Membran umfasst, ermöglicht eine Abtrennung des Wasserstoffs aus dem gasförmigen Produktstrom der ersten Separatorstufe. Die dritte Membranstufe wird bevorzugt bei Umgebungstemperatur von etwa 10°C bis etwa 50°C, wie etwa 20 bis etwa 30°C betrieben. Der Wasserstoff wird zum Eingang des Fischer-Tropsch Reaktors zurückgeführt.

Eine anschließende Tieftemperaturrektifikation kann durch die erfindungsgemäße Verschaltung von Membran- und Kondensationsstufen erheblich entlastet werden, da hierdurch bereits eine Vorfraktionierung realisiert wird.

Gemäß einer weiteren Ausführungsform der vorliegenden Erfindung wird der leichte Gase enthaltende Permeatstrom aus der dritten Membranstufe einer vierten Membranstufe zur Bildung eines leichte Gase enthaltenden Retentatstroms und eines an C₂₊-Kohlenwassertoffen angereicherten Permeatstroms zugeführt und der leichte Gase enthaltende Retentatstrom der vierten Membranstufe wird bevorzugt mit dem Einlaßstrom zur Rückführung in die erste Membranstufe gemischt.

Gemäß einer weiteren Ausführungsform der vorliegenden Erfindung wird der Permeatstrom der vierten Membranstufe einer fünften Membranstufe zur Bildung eines Kohlendioxid enthaltenden Permeatstroms und eines Kohlenwasserstoffe enthaltenden Retentatstroms der fünften Membranstufe zugeführt.

Gemäß einer weiteren Ausführungsform der vorliegenden Erfindung wird mindestens eine flüssige Fraktion der ersten Separatorstufe einer zweiten Separatorstufe zur Erzeugung mindestens einer schwereren Fraktion und einer leichteren Fraktion zugeführt. Die schwerere Fraktion ist vorzugsweise eine C₅₊, bevorzugter eine C₇₊, besonders bevorzugt eine C₁₀₊-Fraktion. Die leichtere Fraktion ist bevorzugt eine C₆₋, bevorzugter eine C₄₋-Fraktion, je nach Dampf-Flüssig-Gleichgewicht. Die schwerere Fraktion der zweiten Separatorstufe kann zur Bildung von unterschiedlichen Brennstoffen, wie Benzin, Fulgbenzin, Diesel, Schiffsdiesel oder Wachsen weiter fraktioniert werden. Die leichtere Fraktion der Separatorstufe kann mit dem Permeatstrom der vierten Membransstufe gemischt werden.

Eine bevorzugte Membran der ersten Membranstufe umfasst eine Poly(ethylenoxid)-Terephthalat-Blockcopolymer-Membran und/oder eine Polyether-Polyamid-Blockcopolymer-Membran.

Eine bevozugte Membran der zweiten Membranstufe umfasst eine Polyimidmembran.

Eine bevozugte Membran der dritten Membranstufe umfasst eine Polyimidmembran.

Eine bevozugte Membran der vierten Membranstufe umfasst eine Siloxanmembran.

Eine bevorzugte Membran der fünften Membranstufe umfasst eine Polyimidmembran umfasst.

### Detaillierte Beschreibung der Erfindung

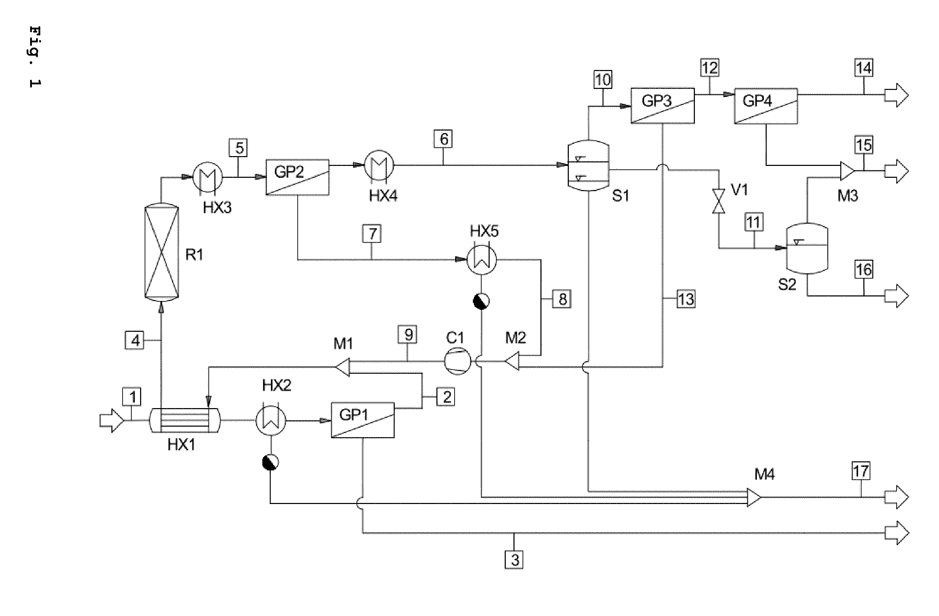

Die Erfindung wird beispielhaft anhand der angefügten Figuren 1 bis 3 erläutert, die das erfindungsgemäße Fischer-Tropsch-Verfahren zur Herstellung von Kohlenwasserstoffen aus Biogas schematisch, beispielhaft erläutern. In den angefügten Figuren bezeichnen:
**C1:** Verdichter
**GP1:** Gaspermeationsstufe ausgestattet mit einer kohlendioxid- und wasserdampfselektiven Membran
**GP2** und **GP3:** Gaspermeationsstufen ausgestattet mit einer wasserstoff- und wasserdampfselektiven Membran
**GP4:** Gaspermeationsstufe ausgestattet mit einer Membran, die sich durch eine hohe Selektivität für höhere Kohlenwasserstoffe auszeichnet.
**GP5:** Gaspermeationsstufe ausgestattet mit einer kohlendioxidselektiven Membran
**HX1** bis **HX5:** Apparate zum Wärmeaustausch und zur Kühlung
**M1** bis **M5:** Gasphasenmischer
**R1:** Fischer-Tropsch Reaktor
**S1:** Drei-Phasen Separator
**S2:** Zwei-Phasen Separator
**V1:** Entspannungsventil
**1** bis **18:** Nummerierung der die Apparate verbindenden Stoffströme.

Bevorzugte Stoffmengenströme, Zusammensetzungen, Drücke, Temperaturen und Phasenzustand der Ströme sind in der ebenfalls angefügten Tabelle 1 aufgeführt.

Figur 1 zeigt beispielhaft ein erfindungsgemäßes Fischer-Tropsch-Verfahren zur Herstellung von Kohlenwasserstoffen aus Synthesegas aus der Biomassenvergasung oder aus einem anderen Synthesegas. Dem Verfahren wird ein aus einer Biomassenvergasung stammendes Synthesegas als Einsatzstrom **1** zugeführt.

Eine typische Zusammensetzung eines aus einer Biomassenvergasung stammenden Synthesegases ist in K.-W. Jun et al., Applied Catalysis A: General 259 (2004) 221-226 und M.J.A. Tijmensen et al., Biomass and Bioenergy 23 (2002) 129-152 beschrieben.

Um einen hohen Umsatz zu den gewünschten Produkten in einer heterogen katalysierten Fischer-Tropsch Synthese zu erreichen, sollte das Verhältnis n H2/(2n CO + 3n C02) möglichst etwa 1 betragen, wobei n H2, n CO und n C02 die Einsatzstoffmengenströme der Komponenten Wasserstoff, Kohlenmonoxid und Kohlendioxid für den Fischer-Tropsch-Reaktor R1 sind. In einem typischen, aus der Biomassenvergasung stammenden Synthesegas beträgt dieses Verhältnis jedoch nur 0,43. Dies ist vor allem einem zu hohen Kohlendioxidgehalt geschuldet.

Das gewünschte Verhältnis n H2/(2n CO + 3n CO2) wurde bisher durch Wassergasshiftreaktion eingestellt, in der Wasserstoff und Kohlendioxid zu Wasserdampf und Kohlenmonoxid umgesetzt werden, wobei der für das Fischer-Tropsch-Verfahren wertvolle Wasserstoff chemisch teilweise umgesetzt wird und für die Fischer-Tropsch-Reaktion hinderlicher, zusätzlicher Wasserdampf erzeugt wird. Erfindungsgemäß wird Synthesegasverhältnis durch gezielte Ausschleusung von Kohlendioxid in einer ersten Membranstufe GP1 (Gaspermeationsstufe 1), die mit einer Membran ausgestattet ist, die eine Selektivität für Kohlendioxid und Wasserdampf besitzt, eingestellt und der Einsatzstrom zur Bildung eines getrockneten Einsatzstroms getrocknet.

Die Membranstufe GP1 ist vorzugsweise mit einer Mehrschichtkompositmembran ausgestattet, welche eine aktive Trennschicht besitzt, die sich durch hohe Kohlendioxid- und Wasserdampfpermeanzen sowie eine gute Selektivität dieser beiden Komponenten gegenüber den anderen im Synthesegas enthaltenen Komponenten ausweist. Beispielhaft für eine solche, erfindungsgemäß verwendbare Mehrschichtkompositmembran ist eine Mehrschichtkompositmembran, die eine aktive Trennschicht aus einem Poly(ethylenoxid)-Terephthalat-Blockcopolymer, wie einem Poly(ethylenoxid)-Terephthalat/Poly(butylenoxid)-Terephthalat-Blockcoplymer umfasst. Ein solches Blockcopolymer ist beispielsweise unter dem Handelsnamen PolyActive9 erhältlich.

Alternativ ist für die erste Membranstufe eine Membran verwendbar, die ein Polyether-Polyamid-Blockcopolymer als aktive trennschicht umfasst. Ein solches Polyether-Polyamid-Blockcopolymer ist beispielsweise unter dem Handelsnamen PEBAX^{®} erhältlich.

Erfindungsgemäß verwendbare Membranen für die erste Membranstufe sind beispielsweise in A. Car et al. Adv. Func. Mater. 18 (2008) 2815-2823, T. Brinkmann et al. Separation of CO2 from Biogas by Gas Permeation, Proceedings 13th Aachener Membrankolloquium, 27.-28. October 2010, Aachen beschrieben. Beispielsweise ist sei eine Mehrschichtmembran erfindungsgemäß verwendbar, die ein Vliesmaterial aus Polyester, Polyphenylensulfid oder Polypropylen umfaßt, auf das eine poröse Stützstruktur aus z.B. Polyacrylnitril oder Polyetherimid aufgebracht ist. Darauf können wiederum eine oder mehrere dichte Polymerschichten als trennaktive Schichten aufgebracht, wie z.B. eine erste Draina geschicht aus Polydimethylsiloxan, gefolgt von der erwähnten PolyActive^{®} oder PEBAX^{®} Schicht als aktiver Trennschicht, wiederum gefolgt von einer weiteren PDMS Schicht als Schutzschicht.

Vorzugsweise wird die Membran der Membranstufe **GP1** bei einer Temperatur im Bereich von 5°C bis 50°C, beispielsweise von 20°C bis 30°C, insbesondere etwa 25°C betrieben. Dazu wird der Einsatzstrom vorzugsweise durch die Wärmeaustauscher **HX1** und/oder **HX2** auf die gewünschte Temperatur gekühlt. Die Verwendung von Wärmetauschern hat den zusätzlichen Vorteil, dass bereits ein Großteil der Wasserfracht entfernt werden kann. Weiterhin kann die durch die Wärmeaustauscher erzeugte Wärme zur Aufheizung des Reaktoreinsatzstromes **4** sowie zur Bereitstellung von Prozesswärme, z.B. für die Biomassenvergasung, oder zur Erzeugung von gespanntem Dampf für die energetische Verwertung in einer Dampfturbine genutzt werden.

Üblicherweise enthält der in der Membranstufe **GP1** abgetrennte Permeatgasstrom **3** hauptsächlich Kohlendioxid. Daneben können aber auch noch energetisch hochwertige Gase wie Methan und Wasserstoff vorhanden sein. Diese können beispielsweise zur Generierung von elektrischer Energie mittels eines Gasmotors, einer Gasturbine oder einer Brennstoffzelle verwendet werden. Auch eine Verwendung zur Erzeugung der für die Biomassenvergasung notwendigen Energie ist möglich.

Der in der Membranstufe **GP1** erzeugte gereinigte Einsatzstrom **2** (Retentatstrom) wird bevorzugt im Mischer **M1** mit Rezirkulationsgasströmen gemischt, und dann bevorzugt durch Wärmeaustausch im Wärmeaustauscher **HX1** erwärmt, vorzugsweise auf die Reaktionstemperatur von 200°C bis 400°C, besonders bevorzugt auf etwa 300°C, und dem Fischer-Tropsch-Reaktor **R1** als getrockneter, erwärmter Einsatzstrom **4** zugeführt.

In dem Fischer-Tropsch-Reaktor **R1** wird der getrocknete Einsatzstrom **4** teilweise zu höheren Kohlenwasserstoffen umgesetzt. Typische erfindungsgemäß geeignete Verfahrensbedingungen, Umsätze und Produktverteilungen sind in K.-W. Jun et al., Applied Catalysis A: General 259 (2004) 221-226 offenbart.

Der Produktstrom aus dem Fischer-Tropsch-Reaktor R1 wird vorzugsweise durch den Wärmeaustauscher **HX3** von etwa Reaktionstemperatur (vorzugsweise etwa 200°C bis 400°C) auf eine Temperatur im Bereich von 150°C bis 250°C, vorzugsweise etwa 200°C heruntergekühlt. Die so freigesetzte Wärme kann z.B. für die Vorwärmung des Einsatzstroms **1** aus der Biomassenvergasung verwendet werden. Dieser Produktstrom **5** stellt den Einsatzstrom der zweiten Gaspermeationsstufe **GP2** dar. Diese Stufe ist vorzugsweise mit einer Polyimidmembran, wie einer Matrimidmembran ausgestattet. Als Polyimid weist Matrimid eine hohe Selektivität von Wasserdampf und Wasserstoff gegenüber Kohlenwasserstoffen und Permanentgasen wie Stickstoff, Kohlenmonoxid und Methan auf. Bei Umgebungstemperatur ist die Selektivität von Wasserstoff gegenüber Kohlendioxid jedoch gering. Bei höheren Temperaturen steigt diese Selektivität, was in dieser Membranstufe vorzugsweise ausgenutzt wird. So enthält der Permeatstrom **7** der zweiten Gaspermeationsstufe GP2 hauptsächlich Wasserstoff und Wasserdampf. Eine beispielhafte erfindungsgemäße Membran wird in Shishatskiy at al. Präsentation EuroMembrane 2004, Hamburg, 2004 beschrieben.

Alternativ zu Polyimid kann beispielsweise ein anderes, temperaturbeständiges, glasartiges Polymer mit ähnlichen Eigenschaften verwendet werden.

Das im Permeatstrom 7 der zweiten Gaspermeationsstufe GP2 enthaltene Wasser wird bevorzugt in Wärmeaustauscher HX5 auskondensiert und abgeführt. Das wasserstoffreiche Gas 8 wird be vorzugt im Mischer **M2** mit einem weiteren wasserstoffreichen Rezirkulationsstrom gemischt, im Verdichter **C1** rekomprimiert und als Strom **9** mit dem ursprünglichen, in der Membranstufe **GP1** getrockneten und teilweise von Kohlendioxid befreiten Einsatzstrom **2** gemischt, um dann nach Aufheizung in **HX1** als Einsatzstrom **4** dem Reaktor **R1** zugeführt zu werden.

Der Retentatstrom der zweiten Gaspermeationsstufe **GP2** wird vorzugsweise im Wärmeaustauscher **HX4** auf eine Temperatur von 20°C bis 30°C, wie etwa 25°C abgekühlt. Wie bereits für den Wärmetauscher **HX3** ausgeführt, können die in **HX4** und **HX5** gewonnene Wärme als Prozesswärme verwendet werden.

Der Retentatstrom der zweiten Gaspermeationsstufe **6** wird einer ersten Separatorstufe **S1** zugeführt und in mindestens einen gasförmigen Produktstrom und mindestens einen flüssigen Produktstrom, vorzugsweise in einen gasförmigen Produktstrom, eine organische Flüssigphase und eine wässrige Flüssigphase getrennt. Eine wässrige Flüssigphase kann als Abwasser aus der ersten Separatorstufe **S1** aus dem Verfahren abgeführt werden. Der flüssige organische Produktstrom der ersten Separatorstufe **S1** wird vorzugsweise mittels des Ventils **V1** entspannt, wobei ein zweiphasiger Strom **11** entsteht, der bevorzugt einer zweiten Separatorstufe **S2** zugeführt und getrennt wird. Mindestens ein flüssiger Produktstrom **16** aus der zweiten Separatorstufe **S2** umfasst höhere Kohlenwasserstoffe, wie solche mit einer Anzahl von mindestens 5 Kohlenstoffatomen, bevorzugt mindestens 8 Kohlenstoffatomen, besonders bevorzugt mindestens 10 Kohlenstoffatomen.

Der gasförmige Produktstrom **10** aus der Separatorstufe **S1** wird einer dritten Membranstufe **GP3** zugeführt, die eine wasserstoffselektive und wasserdampfselektive Membran umfasst und in der mindestens ein Teil des in der Kopffraktion vorhandenen Wasserstoffs und Wasserdampfs von leichten Gasen zur Bildung eines hauptsächlich Wasserstoff enthaltenden Permeatstroms der dritten Membranstufe und eines leichte Gase enthaltenden Retentatstroms der dritten Membranstufe getrennt werden. Diese Stufe ist vorzugsweise ebenfalls mit einer wasserstoffselektiven und wasserdampfselektiven Polyimidmembran, wie einer Matrimidmembran ausgestattet. Bei einer bevorzugten Betriebstemperatur von etwa 10°C bis etwa 30°C, wie etwa 25°C wird durch eine Matrimidmembran zusätzlich Kohlendioxid gut aus dem gasförmigen Produktstrom 10 der ersten Separatorstufe S1 abgetrennt. Neben Matrimid kann auch ein anderes glasartitiges Polymer mit ähnlichen Eigenschaften verwendet werden.

Der Wasserstoff und Kohlendioxid enthaltende Permeatstrom 13 der dritten Membranstufe GP3 bevorzugt wird im Mischer M2 mit dem Permeatstrom der zweiten Membranstufe GP2 gemischt und in der oben beschriebenen Weise dem Reaktor R1 wieder zugeführt. Gegebenenfalls noch enthaltener Wasserdampf wird in der Membranstufe GP3 mit dem Permeatstrom 13 aus dem Produktgas entfernt, so dass ein getrockneter Retentatstrom 12 der dritten Membranstufe GP3 der weiteren Gasaufbereitung zugeführt werden kann.

Vorzugsweise findet in einer vierten Membranstufe GP4, die mit einer für höhere Kohlenwasserstoffe selektiven Membran, wie z.B. einer Polyoctylmethylsiloxan-Kompositmembran eine weitere Fraktionierung des Retentatstroms 12 der dritten Membranstufe GP3 statt. Polyoctylmethylsiloxan-Kompositmembran werden beispielsweise in Ohlrogge, K., J. Wind, and T. Brinkmann, Membranes for the recovery of volatile organic compounds in Comprehensive membrane science and engineering, E. Drioli and L. Giorno, Editors. 2010, Academic Press (Elsevier): Oxford beschrieben. Diese Fraktionierung kann so eingestellt werden, dass eine möglicherweise folgende Tieftemperatur/Hochdruckgasaufbereitung entlastet wird, z.B. in dem die leichten Gase in dem Reten tatstrom 14 aufkonzentriert werden und einer energetischen Verwendung wie z.B. einer Gasturbine zugeführt werden. Dabei ist eine Mischung mit dem der Membranstufe GP1 entnommenen Permeatstrom 3 möglich.

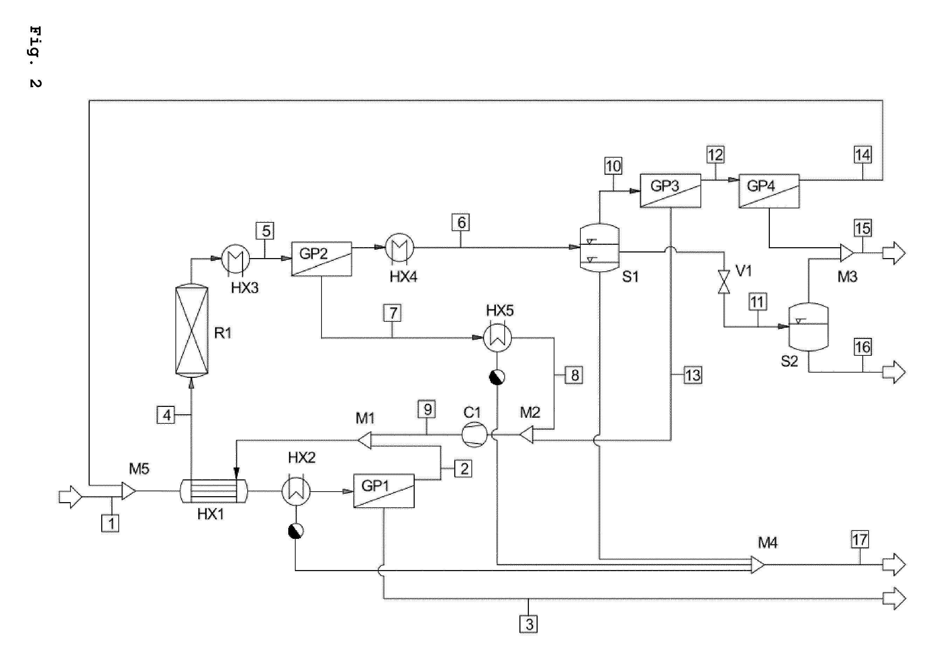

Es ist auch möglich den Retentatstrom 14 der vierten Membranstufe GP4 wieder dem Einsatzstrom 1 beizumischen, um so die Ausbeute des Verfahrens zu erhöhen. Eine solche bevorzugte Ausführungsform der Erfindung ist in Figur 2 dargestellt.

Der in der vierten Membranstufe GP4 verwendete Membranwerkstoff sollte sich durch eine hohe Selektivität höherer Kohlenwasserstoffe gegenüber leichter Gase wie Methan, Kohlenmonoxid, Stickstoff oder Wasserstoff auszeichnen. Besonders bevorzugt werden für die vierte Membranstufe Siloxanpolymere verwendet. Beispielhafte Membranmaterialien umfassen Polyoctylmethylsiloxan und Polydimethylsiloxan. Der Retentatstrom der vierten Membranstufe enthält vorzugsweise hochwertige Grundstoffe für die petrochemische Industrie wie z.B. Propan und Propylen.

Der Permeatstrom der Membranstufe GP4 wird bevorzugt mit dem diese Komponenten ebenfalls enthaltenden gasförmigen Produktstrom des zweiten Separators S2 im Mischer M3 gemischt und stellt so einen weiteren Produktstrom des Verfahrens dar, welcher dann der oben beschriebenen weiteren Gasaufbereitung zugeführt wird. Eine beispielhafte Gasaufbereitung wird z.B. in G. Hochgesand, Erdgas Aufbereitung, in Ullmanns Enzyklopädie der technischen Chemie. 1975, VCH: Weinheim beschrieben.

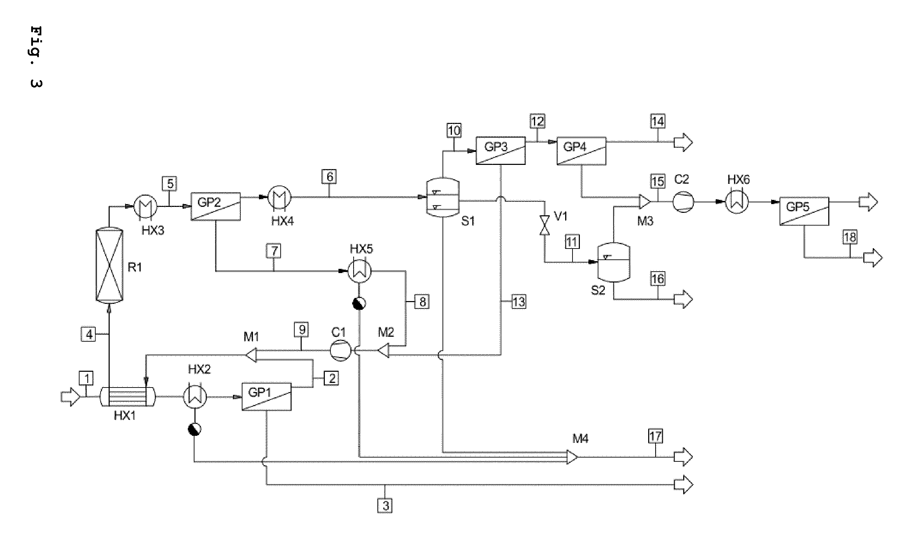

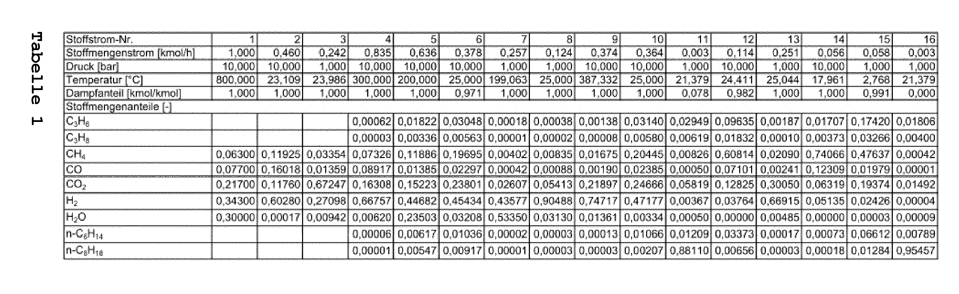

Es ist jedoch möglich, das im Strom 15 enthaltene Kohlendioxid durch ein fünfte Membranstufe GP5, die mit eine kohlendioxidselektiven Membran, wie einer Polyimidmembran ausgestattet ist, ganz oder teilweise zu entfernen. Eine solche bevorzugte Ausführungsform der Erfindung ist in Figur 3 schematisch wiedergegeben. Für die fünfte Membranstufe **GP5** wird bevorzugt eine Matrimid- oder eine PolyActive-Membran oder eine andere CO₂-selektiven glas- oder gummiartigen Polymermembran verwendet.

Das Permeatgas **18** dieser Stufe kann wiederum rezirkuliert werden. Das Abwasser des Verfahrens wird im Mischer M4 gesammelt und als Strom (17) abgeführt.

## Patentansprüche

1. Verfahren zur Herstellung von Kohlenwasserstoffen aus Biomasse, bei dem ein Einsatzstrom (1) aus der Biomassenvergasung oder aus einem anderen Synthesegaserzeungungsverfahren, der Wasserstoff, Kohlenmonoxid sowie weitere Gase und/oder Dämpfe umfasst, zunächst einer ersten Membranstufe (GP1) zugeführt wird, die eine wasserdampfselektive und kohlendioxidselektive Membran umfasst und durch die mindestens ein Teil des in dem Einsatzstrom vorhandenen Wasserdampfs und ein Teil des im Einsatzstroms vorhandenes Kohlendioxid vom Einsatzstrom zur Bildung eines Wasserdampf und Kohlendioxid umfassenden Permeatstroms (3) der ersten Membranstufe und eines Wasserstoff und Kohlenmonoxid enthaltenden Retentatstroms (2) der ersten Membranstufe getrennt wird, der Retentatstrom (2) der ersten Membranstufe einem Fischer-Tropsch-Reaktor (R1) zugeführt wird, in dem ein Produktstrom (5) erzeugt wird, der Wasserstoff, Wasserdampf, Kohlenwasserstoffe sowie gegebenenfalls weitere Gase und/oder Dämpfe umfasst, der Produktstrom (5) aus dem Fischer-Tropsch-Reaktor (R1) einer zweiten Membranstufe (GP2) zugeführt wird, die eine wasserstoffselektive und wasserdampfselektive Membran umfasst und durch die mindestens ein Teil des im Produktstrom (5) des Fischer-Tropsch-Reaktors (R1) vorhandenen Wasserstoffs und Wasserdampfs zur Bildung eines Wasserstoff und Wasserdampf enthaltenden Permeatstroms (7) der zweiten Membranstufe (GP2) und eines Kohlenwasserstoffe enthaltenden Retentatsstroms (6) der zweiten Membranstufe getrennt werden, der Permeatstrom (7) der zweiten Membranstufe (GP2) mit dem Retentatstrom (2) der ersten Membranstufe (GP1) gemischt und dem Reaktor (R1) wieder zugeführt wird, der Retentatsstrom (6) der zweiten Membranstufe einer ersten Separatorstufe (S1) zugeführt wird, in der der Retentatsstrom (6) der zweiten Membranstufe mindestens in Gasphase (10) und eine flüssige Fraktion sowie gegebenenfalls in weitere Fraktionen aufgetrennt wird, die Gasphase (10) der ersten Separatorstufe (S1) einer dritten Membranstufe (GP3) zugeführt wird, die eine wasserstoffselektive und wasserdampfselektive Membran umfasst und in der mindestens ein Teil des in der Gasphase (10) der ersten Separatorstufe (S1) vorhandenen Wasserstoffs von leichten Gasen zur Bildung eines Wasserstoff und Wasserdampf enthaltenden Permeatstroms (13) der dritten Membranstufe (GP3) und eines leichte Gase enthaltenden Retentatstroms (12) der dritten Membranstufe (GP3) getrennt werden, und der Wasserstoff enthaltende Permeatstrom (13) der dritten Membranstufe (GP3) mit dem Retentatstrom (2) der ersten Membranstufe (GP1) und dem Permeatstrom (7) der zweiten membranstufe (GP2) zur Rückführung in den Fischer-Tropsch-Reaktor (R1) gemischt wird.

2. Verfahren nach Anspruch 1, bei dem mindestens ein Teil des in dem Einsatzstrom (1) vorhandenen Wasserdampfs und ein Teil des im Einsatzstroms vorhandenes Kohlendioxid vom Einsatzstrom zur Bildung eines eines Wasserdampf und Kohlendioxid umfassenden Permeatstroms (3) und eines Wasserstoff und Kohlenmonoxid enthaltenden Retentatstroms (2) in der ersten Membranstufe (GP1) bei Temperaturen von 10°C bis 50°C erfolgt getrennt wird.

3. Verfahren nach einem der Ansprüche 1 oder 2, bei dem mindestens ein Teil des im Produktstrom (5) des Fischer-Tropsch-Reaktors (R1) vorhandenen Wasserstoffs und Wasserdampfs zur Bildung eines Wasserstoff und Wasserdampf enthaltenden Permeatstroms (7) und eines Kohlenwasserstoffe enthaltenden Retentatsstroms (6) in der zweiten Membranstufe (GP2) bei Temperaturen von 75°C bis 250°C getrennt werden.

4. Verfahren nach einem der vorgehenden Ansprüche, bei dem mindestens ein Teil des in der Gasphase (10) der ersten Separatorstufe (S1) vorhandenen Wasserstoffs von leichten Gasen zur Bildung eines Wasserstoff enthaltenden Permeatstroms (13) und eines leichte Gase enthaltenden Retentatstroms (12) in der dritten Membranstufe (GP3) bei einer Temperatur von 10°C bis 50°C getrennt werden.

5. Verfahren nach einem der vorgehenden Ansprüche, bei dem der leichte Gase enthaltende Retentatstrom (12) aus der dritten Membranstufe (GP3) einer vierten Membranstufe (GP4) zur Bildung eines leichte Gase enthaltenden Retentatstroms (14) und eines an C2+ Kohlenwasserstoffen aufkozentrierten Permeatstroms zugeführt wird.

6. Verfahren nach Anspruch 6, bei dem der leichte Gase enthaltende Retentatstrom (14) der vierten Membranstufe (GP4) mit dem Einlaßstrom (1) zur Rückführung in die erste Membranstufe (GP1) gemischt wird.

7. Verfahren nach einem der vorgehenden Ansprüche, bei dem der Permeatstrom der vierten Membranstufe einer fünften Membranstufe (GP5) zur Bildung eines Kohlendioxid enthaltenden Permeatstroms (18) und eines Kohlenwasserstoffe enthaltenden Retentatstroms der fünften Membranstufe zugeführt wird.

8. Verfahren nach einem der vorgehenden Ansprüche, bei dem mindestens eine flüssige Fraktion (11) der ersten Separatorstufe (S1) einer zweiten Separatorstufe (S2) zur Trennung mindestens einer schwereren Fraktion (16) und einer leichteren Fraktion zugeführt wird.

9. Verfahren nach Anspruch 8, bei dem die leichtere Fraktion der zweiten Separatorstufe (S2) mit dem Permeatstrom der vierten Membransstufe (GP4) gemischt wird.

10. Verfahren nach einem der vorgehenden Ansprüche, bei dem die Membran der ersten Membranstufe (GP1) eine Poly(ethylenoxid)-Terephthalat-Blockcopolymer-Membran und/oder eine Polyether-Polyamid-Blockcopolymer-Membran umfasst.

11. Verfahren nach einem der vorgehenden Ansprüche, bei dem die Membran der zweiten Membranstufe (GP2) eine Polyimidmembran umfasst.

12. Verfahren nach einem der vorgehenden Ansprüche, bei dem die Membran der dritten Membranstufe (GP3) eine Polyimidmembran umfasst.

13. Verfahren nach einem der Ansprüche 5 bis 11, bei dem die Membran der vierten Membranstufe (GP4) eine Siloxanmembran ist.

14. Verfahren nach einem der Ansprüche 7 bis 13, bei dem die Membran der fünften Membranstufe (GP5) eine Polyimidmembran umfasst.

15. Verfahren nach einem der vorgehenden Ansprüche, bei dem der Permeatstrom (7) der zweiten Mambranstufe (GP2) durch Kondensation in einem Wärmetauscher (HX5) weitgehend von Wasser befreit wird, in einem Mischer (M2) mit einem weiteren Rezirkulationsstrom (13) vermischt, in einem Verdichter (C1) verdichtet wird, bevor er mit dem Retentatstrom (2) der ersten Membranstufe (GP1) gemischt wird.

## Claims

1. Process for producing hydrocarbons from biomass in which an input stream (1) from biomass gasification or another synthesis gas production process which comprises hydrogen, carbon monoxide as well as further gases and/or vapours is initially fed to a first membrane stage (GP1) which comprises a steam-selective and carbon dioxide-selective membrane and through which at least some of the steam present in the input stream and some of the carbon dioxide present in the input stream are separated from the input stream to form a permeate stream (3) of the first membrane stage containing hydrogen and carbon monoxide and a retentate stream (2) of the first membrane stage containing hydrogen and carbon monoxide, the retentate stream (2) of the first membrane stage is fed to a Fischer-Tropsch reactor (R1) in which a product stream (5) is produced which comprises hydrogen, steam, hydrocarbons as well as optionally further gases and/or vapours, the product stream (5) from the Fischer-Tropsch reactor (R1) is fed to a second membrane stage (GP2) which comprises a hydrogen-selective and steam-selective membrane and through which at least some of the hydrogen and steam present in the product stream (5) of the Fischer-Tropsch reactor (R1) are separated to form a permeate stream (7) of the second membrane stage (GP2) containing hydrogen and steam and a retentate stream (6) of the second membrane stage containing hydrocarbons, the permeate stream (7) of the second membrane stage (GP2) is mixed with the retentate stream (2) of the first membrane stage (GP1) and fed back to the reactor (R1), the retentate stream (6) of the second membrane stage is fed to a first a first separator stage (S1) in which the retentate stream (6) of the second membrane stage is separated at least into a gas phase (10) and liquid fraction, as well as optionally into further fractions, the gas phase (1) of the first separator stage (S1) is fed to a third membrane stage (GP3) which comprises a hydrogen-selective and steam-selective membrane and in which at least some of the hydrogen present in the gas phase (10) of the first separator stage (S1) is separated from light gases to form a permeate stream (13) of the third membrane stage (GP3) containing hydrogen and steam and a retentate stream (12) of the third membrane stage (GP3) containing light gases, and the permeate stream (13) of the third membrane stage (GP3) containing hydrogen is mixed with the retentate stream (2) of the first membrane stage (GP1) and the permeate stream (7) of the second membrane stage (GP2) for recirculation into the Fischer-Tropsch reactor (R1).

2. Process according to claim 1, in which at least some of the steam present in the input stream (1) and some of the carbon dioxide present in the input stream are separated from the input stream to form a permeate stream (3) comprising steam and carbon dioxide and a retentate stream (2) containing hydrogen and carbon monoxide in the first membrane stage (GP1) at temperatures of from 10°C to 50°C.

3. Process according to one of claims 1 or 2, in which at least some of the hydrogen and steam present in the product stream (5) of the Fischer-Tropsch reactor (R1) are separated to form a permeate stream (7) containing hydrogen and steam and a retentate stream (6) containing hydrocarbons in the second membrane stage (GP2) at temperatures of from 75°C to 250°C.

4. Process according to one of the previous claims, in which at least some of the hydrogen present in the gas phase (10) of the first separator stage (S1) is separated from light gases to form a permeate stream (13) containing hydrogen and a retentate stream (12) containing light gases in the third membrane stage (GP3) at a temperature of from 10°C to 50°C.

5. Process according to one of the previous claims, in which the retentate stream (12) containing light gases from the third membrane stage (GP3) is fed to a fourth membrane stage (GP4) to form a retentate stream (14) containing light gases and a permeate stream enriched with C2+ hydrocarbons.

6. Process according to claim 5, in which the retentate stream (14) of the fourth membrane stage (GP4) containing light gases is mixed with the input stream (1) for recirculation into the first membrane stage (GP1).

7. Process according to one of the previous claims, in which the permeate stream of the fourth membrane stage is fed to a fifth membrane stage (GP5) to form a permeate stream (18) containing carbon dioxide and a retentate stream of the fifth membrane stage containing hydrocarbons.

8. Process according to one of the previous claims, in which at least one liquid fraction (11) of the first separator stage (S1) is fed to a second separator stage (S2) to separate at least one heavier fraction (16) and a lighter fraction.

9. Process according to claim 8, in which the lighter fraction of the second separator stage (S2) is mixed with the permeate stream of the fourth membrane stage (GP4).

10. Process according to one of the previous claims, in which the membrane of the first membrane stage (GP1) comprises a poly(ethylene oxide)-terephthalate block copolymer membrane and/or a polyether-polyamide block copolymer membrane.

11. Process according to one of the previous claims, in which the membrane of the second membrane stage (GP2) comprises a polyimide membrane.

12. Process according to one of the previous claims, in which the membrane of the third membrane stage (GP3) comprises a polyimide membrane.

13. Process according to one of claims 5 to 11, in which the membrane of the fourth membrane stage (GP4) is a siloxane membrane.

14. Process according to one of claims 7 to 13, in which the membrane of the fifth membrane stage (GP5) comprises a polyimide membrane.

15. Process according to one of the previous claims, in which water is largely removed from the permeate stream (7) of the second membrane stage (GP2) by condensation in a heat exchanger (HX5), said permeate stream is mixed in a mixer (M2) with a further recirculation stream (13), compressed in a compressor (C1), before it is mixed with the retentate stream (2) of the first membrane stage (GP1).

## Revendications

1. Procédé de fabrication d'hydrocarbures à partir de biomasse, selon lequel :
un flux de charge (1) issu de la gazéification de biomasse ou d'un autre procédé de génération de gaz de synthèse, qui comporte de l'hydrogène, du monoxyde de carbone et d'autres gaz et/ou vapeurs, est d'abord amené à un premier étage à membrane (GP1) qui comprend une membrane sélective de vapeur d'eau et sélective de dioxyde de carbone et par lequel au moins une partie de la vapeur d'eau présente dans le flux de charge et une partie du dioxyde de carbone présent dans le flux de charge sont séparées du flux de charge, en vue de former un flux de perméat (3) du premier étage à membrane, contenant de la vapeur d'eau et du dioxyde de carbone, et un flux de rétentat (2) du premier étage à membrane, contenant de l'hydrogène et du monoxyde de carbone; le flux de rétentat (2) du premier étage à membrane est amené à un réacteur Fischer-Tropsch (R1) dans lequel est généré un flux de produit (5) qui comporte de l'hydrogène, de la vapeur d'eau, des hydrocarbures et, le cas échéant, d'autres gaz et/ou vapeurs; le flux de produit (5) issu du réacteur Fischer-Tropsch (R1) est amené à un deuxième étage à membrane (GP2) qui comprend une membrane sélective d'hydrogène et sélective de vapeur d'eau et par lequel au moins une partie de l'hydrogène et de la vapeur d'eau présents dans le flux de produit (5) du réacteur Fischer-Tropsch (R1) est séparée en vue de former un flux de perméat (7) du deuxième étage à membrane (GP2), contenant de l'hydrogène et de la vapeur d'eau, et un flux de rétentat (6) du deuxième étage à membrane, contenant des hydrocarbures; le flux de perméat (7) du deuxième étage à membrane (GP2) est mélangé avec le flux de rétentat (2) du premier étage à membrane (GP1) et est ramené au réacteur (R1); le flux de rétentat (6) du deuxième étage à membrane est amené à un premier étage de séparateur (S1) dans lequel le flux de rétentat (6) du deuxième étage à membrane est séparé au moins en une phase gazeuse (10) et une fraction liquide et, le cas échéant, en d'autres fractions; la phase gazeuse (10) du premier étage de séparateur (S1) est amenée à un troisième étage à membrane (GP3) qui comprend une membrane sélective d'hydrogène et sélective de vapeur d'eau et dans lequel au moins une partie de l'hydrogène présent dans la phase gazeuse (10) du premier étage de séparateur (S1) est séparée des gaz légers, en vue de former un flux de perméat (13) du troisième étage à membrane (GP3), contenant de l'hydrogène et de la vapeur d'eau, et un flux de rétentat (12) du troisième étage à membrane (GP3), contenant des gaz légers; et le flux de perméat (13) du troisième étage à membrane (GP3), contenant de l'hydrogène, est mélangé avec le flux de rétentat (2) du premier étage à membrane (GP1) et le flux de perméat (7) du deuxième étage à membrane (GP2), en vue du retour dans le réacteur Fischer-Tropsch (R1).

2. Procédé selon la revendication 1, selon lequel au moins une partie de la vapeur d'eau présente dans le flux de charge (1) et une partie du dioxyde de carbone présent dans le flux de charge sont séparées du flux de charge en vue de former un flux de perméat (3), contenant de la vapeur d'eau et du dioxyde de carbone, et un flux de rétentat (2) contenant de l'hydrogène et du monoxyde de carbone, dans le premier étage à membrane (GP1), à des températures allant de 10 °C à 50 C.

3. Procédé selon une des revendications 1 ou 2, selon lequel au moins une partie de l'hydrogène et de la vapeur d'eau présents dans le flux de produit (5) du réacteur Fischer-Tropsch (R1) sont séparées en vue de former un flux de perméat (7), contenant de l'hydrogène et de la vapeur d'eau, et un flux de rétentat (6) contenant des hydrocarbures, dans le deuxième étage à membrane (GP2), à des températures allant de 75 °C à 250 °C.

4. Procédé selon une des revendications précédentes, selon lequel au moins une partie de l'hydrogène présent dans la phase gazeuse (10) du premier étage de séparateur (S1) est séparée des gaz légers en vue de former un flux de perméat (13), contenant de l'hydrogène, et un flux de rétentat (12) contenant des gaz légers, dans le troisième étage à membrane (GP3), à une température allant de 10 °C à 50 °C.

5. Procédé selon une des revendications précédentes, selon lequel le flux de rétentat (12) contenant des gaz légers est amené du troisième étage à membrane (GP3) à un quatrième étage à membrane (GP4) en vue de former un flux de rétentat (14), contenant des gaz légers, et un flux de perméat concentré en hydrocarbures C2+.

6. Procédé selon la revendication 6, selon lequel le flux de rétentat (14), contenant des gaz légers, du quatrième étage à membrane (GP4) est mélangé avec le flux d'admission (1) en vue du retour dans le premier étage à membrane (GP1).

7. Procédé selon une des revendications précédentes, selon lequel le flux de perméat du quatrième étage à membrane est amené à un cinquième étage à membrane (GP5) en vue de former un flux de perméat (18), contenant du dioxyde de carbone, et un flux de rétentat contenant des hydrocarbures du cinquième étage à membrane.

8. Procédé selon une des revendications précédentes, selon lequel au moins une fraction liquide (11) du premier étage de séparateur (S1) est amenée à un deuxième étage de séparateur (S2) en vue de séparer au moins une fraction plus lourde (16) et une fraction plus légère.

9. Procédé selon la revendication 8, selon lequel la fraction plus légère du deuxième étage de séparateur (S2) est mélangée avec le flux de perméat du quatrième étage à membrane (GP4).

10. Procédé selon une des revendications précédentes, selon lequel la membrane du premier étage à membrane (GP1) comprend une membrane en copolymère bloc de poly(oxyde d'éthylène)-téréphtalate et/ou une membrane en copolymère bloc de polyéther-polyamide.

11. Procédé selon une des revendications précédentes, selon lequel la membrane du deuxième étage à membrane (GP2) comprend une membrane en polyimide.

12. Procédé selon une des revendications précédentes, selon lequel la membrane du troisième étage à membrane (GP3) comprend une membrane en polyimide.

13. Procédé selon une des revendications 5 à 11, selon lequel la membrane du quatrième étage à membrane (GP4) est une membrane en siloxane.

14. Procédé selon une des revendications 7 à 13, selon lequel la membrane du cinquième étage à membrane (GP5) comprend une membrane en polyimide.

15. Procédé selon une des revendications précédentes, selon lequel le flux de perméat (7) du deuxième étage à membrane (GP2) est débarrassé dans une large mesure de l'eau, par condensation dans un échangeur de chaleur (HX5), est mélangé avec un autre flux de recirculation (13) dans un mélangeur (M2), est comprimé dans un compresseur (C1), avant d'être mélangé avec le flux de rétentat (2) du premier étage à membrane (GP1).
